Europäisches Patentamt

European Patent Office

Office européen des brevets

(11) Publication number: **0 478 778 A1**

## EUROPEAN PATENT APPLICATION
## published in accordance with Art. 158(3) EPC

(21) Application number: **90909185.2**

(22) Date of filing: **14.06.90**

(86) International application number: **PCT/JP90/00776**

(87) International publication number: **WO 90/15605 (27.12.90 90/29)**

(51) Int. Cl.⁵: **A61K 31/485**, A61K 31/405, A61K 45/00

(30) Priority: **15.06.89 JP 152863/89**

(43) Date of publication of application: **08.04.92 Bulletin 92/15**

(84) Designated Contracting States: **AT BE CH DE DK ES FR GB IT LI LU NL SE**

(71) Applicant: **TAISHO PHARMACEUTICAL CO. LTD**
**24-1 Takata 3-chome Toshima-ku**
**Tokyo 171(JP)**

(72) Inventor: **KASUYA, Yutaka**
**61, Komukainishimachi 1-chome, Saiwai-ku**
**Kawasaki-shi, Kanagawa 210(JP)**
Inventor: **KAMEI, Junzo**
**6-27-101, Yashio 5-chome, Shinagawa-ku**
**Tokyo 140(JP)**
Inventor: **AIHARA, Hironaka, Taisho**
**Pharmaceutical Co., Ltd.**
**24-1, Takata 3-chome, Toshima-ku**
**Tokyo 171(JP)**
Inventor: **KURACHI, Michio, c/o Taisho**
**Pharmaceutical Co. Ltd**
**24-1, Takata 3-chome, Toshima-ku**
**Tokyo 171(JP)**

(74) Representative: **Lamb, John Baxter et al**
**MARKS & CLERK 57-60 Lincoln's Inn Fields**
**London WC2A 3LS(GB)**

(54) **ANTITUSSIVE.**

(57) Commercially available antitussives which are widely used at present are problematic in that they contain dihydrocodeine which is officially designated as a narcotic or methylephedrine which is used as a starting material for a stimulant. An antitussive composition of the invention comprises a combination of an antitussive and L-tryptophan to enhance the antitussive effect. It has a similar antitussive effect even though the content of the antitussive is reduced, and is free from the side effect of aggravating the physical dependence of an antitussive.

TECHNICAL FIELD

The present invention relates to an antitussive composition for enhancing antitussive activity comprising an antitussive agent and L-tryptophan.

BACKGROUND TECHNIQUE

The current cough inhibition drugs which are widely commercially available are the combination drugs comprising several agents. These combination drugs, however, contain dihydrocodeine designated as a narcotic agent and methylephedrine, which is a starting material of the psychostimulants, as antitussive agents. Therefore, there is a need for the development of the antitussive preparation decreasing the antitussive agent content in spite of keeping the equivalent antitussive activity without the occurrance of the side effect by which the plasticity of physical dependence of the antitussive agent is aggravated.

MEANS TO SOLVE THE PROBLEMS

As a result of the research on the effect of L-tryptophan on the antitussive action, the present inventors have found that when used an antitussive agent in combination with L-tryptophan, L-tryptophan enhances the effect of the antitussive agent, and have acoomplished the present invention,

An object of the present invention is to provide an antitussive preparation comprising an antitussive agent and L-tryptophan.

The antitussive agent in the present invention refers to central antitussive agents such as, for example, dihydrocodeine, noscapine and dextromethorphan.

According to the dosage forms of the antitussive composition of the present invention, the antitussive agent and L-tryptophan may be formulated in a single dosage form or individually in separate dosage forms. As the dosage form, there are forms for oral administration such as powders, syrups, tablets and granules, and forms for parenteral administration such as injectional solutions and suppositories, all of which can be prepared using known carriers in the art according to the conventional practices.

Examples of the carriers are fillers (e.g., lactose, flucose, mannitol and crystalline cellulose), disintegraters (e.g., potato starch and carboxymethylcellulose), rubricants (e.g., talc and magnesium stearate), binders (e.g., gelatin, gum arabic, polyvinyl alcohol and hydroxypropylcellulose), all of which can be usually used for preparing the dosage forms for oral administration. If neccessary, corrigents, cosmetics and antiseptics may be added.

The quantity of L-tryptophan used in the antitussive composition may be varied depending on the kind of the antitussive agent and is not critical especially. But there is used preferably from one to 100 parts by weight, and most preferably from 2 to 50 parts by weight of L-tryptophan relative to one part by weight of the antitussive agent.

BEST MODE FOR CARRYING OUT THE INVENTION

The present invention is illustrated in more detail by the following formulations and test examples.
Typical formulations of the antitussive preparations of the present invention are as follows.

Formulation 1

| Dihydrocodeine phosphate | 10 mg |
| L-Tryptophan | 200 mg |
| Crystalline cellulose | 385 mg |
| Corn starch | 385 mg |
| Hydroxypropylcellulose | 20 mg |
| | 1000 mg/pack |

Granules were prepared at the above-mentioned combination ratio.

Formulation 2

| Noscapine hydrochloride | 20 mg |
| L-Tryptophan | 200 mg |
| Corn starch | 760 mg |
| Hydroxypropylcellulose | 20 mg |
| | 1000 mg/capsule |

Capsules were prepared at the above-mentioned combination ratio.

Formulation 3

| Dihydrocodeine phosphate | 20 mg |
| L-Tryptophan | 100 mg |
| Mannitol | 400 mg |
| Low substituted hydroxypropylcellulose | 460 mg |
| Polyvinyl pyrrolidone | 20 mg |
| | 1000 mg/pack |

Granules were prepared at the above-mentioned combination ratio.

Formulation 4

|  |  |
|---|---|
| Dihydrocodeine phosphate | 20 mg |
| L-Tryptophan | 100 mg |
| White suger | 430 mg |
| Low substituted hydroxypropylcellulose | 430 mg |
| Polyvinyl pyrrolidone | 20 mg |
|  | 1000 mg/pack |

Granules were prepared at the above-mentioned combination ratio.

Formulation 5

|  |  |
|---|---|
| Dihydrocodeine phosphate | 10 mg |
| L-Tryptophan | 50 mg |
| Mannitol | 122 mg |
| Carboxymethylcellulose calcium | 100 mg |
| Polyvinyl pyrrolidone | 15 mg |
| Stearate magnesium | 3 mg |
|  | 300 mg/tablet |

Tablets were prepared at the above-mentioned combination ratio.

Formulation 6

| | |
|---|---|
| Dihydrocodeine phosphate | 10 mg |
| L-Tryptophan | 20 mg |
| Crystalline cellulose | 152 mg |
| Carboxymethylcellulose calcium | 100 mg |
| Polyvinyl pyrrolidone | 15 mg |
| Stearate magnesium | 3 mg |

300 mg/tablet

Tablets were prepared at the above-mentioned combination ratio.

Formulation 7

| | |
|---|---|
| Dihydrocodeine phosphate | 10 mg |
| L-Tryptophan | 50 mg |
| dl-Methylephedrine hydrochloride | 25 mg |
| Noscapine | 20 mg |
| Carbinoxamine maleate | 4 mg |
| Lysozyme chloride | 20 mg |
| Crystalline cellulose | 500 mg |
| Light silicic acid anhydride | 50 mg |
| Hydroxypropylcellulose | 321 mg |

1000 mg/pack

A powder was prepared at the above-mentioned combination ratio.

## Formulation 8

| | |
|---|---|
| Bromhexine hydrochloride | 12 mg |
| Lysozyme chloride | 60 mg |
| Dihydrocodeine phosphate | 15 mg |
| L-Tryptophan | 75 mg |
| dl-Methylephedrine hydrochloride | 75 mg |
| Carbinoxamine maleate | 12 mg |
| D-solbitol 70 | 20 g |
| Maltitol | 10 g |
| Sodium citrate | 10 mg |
| Purified water | q.s. |
| | 60 ml |

A syrup was prepared at the above-mentioned combination ratio.

The enhancing effect of antitussive action of the present invention is illustrated by the following test examples.

Test Example 1

(Test animals)

Male SD strain rats weighing 250 - 350 g anesthesized by the intraperitoneal administration of 70 mg/kg of $\alpha$-chloralose were used.

(Drugs used)

Dihydrocodeine phosphate (abbreviated as DC)
Methylephedrine hydrochloride (abbreviated as ME)
Noscapine hydrocloride (abbreviated as NC)
L-Tryptophane (abbreviated as L-Trp)

The drugs were administered intravenously (i.v.) or intraduodenally (i.d.).

(Test method)

The test was carried out according to a method similar to that of Kamei, Ogawa and Kasuya in Arch. int. Pharmacodyn., vol. 290, pp. 117 - 127 (1987), Eur. J. Pharmacol., vol. 153, pp. 305 - 308 (1988) and Pharmacol. Biochem. Behav., vol. 34, pp. 595 - 598 (1989) on the whole.

Rats were fixed in the dorsal position, and the cough reflex was induced by giving the electrical stimulation to the tracheal mucosa according to the pancture electrode method. The trochea was exposed by incision in the cervical midline, and the pancture electrode (0.2 mm in diameter and 10 cm in length) which was made of a stainless steel wire coated with epoxy resin for insulation was inserted into the trachea through a gaiding cannula (injection needle 23G). The electrical stimulation was carried out using an electrical stimulation apparatus through an isolator under the conditions with 1.0 msec of a rectangular-wave pulse with a frequence of 40 Hz on 2.0 - 4.0 volts for a 10 sec period of stimuration time. Furthermore, in order to administer the drug, a polypropylene cannula which was filled with physiological saline was inserted into the right femoral artery.

(Test Schedule)

Prior to administration of the drug, the stimulation was carried out for the five minutes intervals in order to obtain the constant cough reflex for the control. Then, the stimulation was repeated at 5, 10, 15, 30, 45 and 60 minutes after administration of the drugs.

(Result)

The antitussive effect was judged as possitive when no cough was induced at all, and as negative when the cough was induced in all 6 stimuli. Six or seven animals were used for each dose, and the reaction rate was obtained by dividing the number of the rats to be inhibited cough by the number of the rats used, and the 50% antitussive dose (AtD50) of each drug was obtained according to the method of Lichfield-Wilcoxon. Results were shown in Table 1.

Table 1

| Effect of L-tryptophan on the antitussive action of dihydrocodeine in rats | | |
|---|---|---|
| Test drug | AtD50[*] (mg/kg) | |
| | (i.v.) | (i.d.) |
| DC<br>DC:L-Trp (1:5) | 0.43 (0.26-0.17)[**]<br>0.20 (0.15-0.26) | 1.83 (1.16-2.90)<br>0.73 (0.47-1.15) |

\* 50% antitussive dose
\*\* 95% confidence limit

When used NC and DM, which are non-narcotic antitussive agents, in combination with L-Trp respectivecy, the resulting AtD50's of NC and DM were shown in Tables 2 and 3. The antitussive effects of NC and DM in combination with L-Trp were each about twice as strong as those of these agents alone like DC which is a narcotic agent.

Table 2

| Effect of L-tryptophan on the antitussive action of noscapine in rats | |
|---|---|
| Test drug | AtD50[*] (mg/kg, i.v.) |
| NC<br>NC:L-Trp (1:5) | 1.06 (0.80-1.51)[**]<br>0.61 (0.42-0.88) |

\* 50% antitussive dose
\*\* 95% confidence limit

Table 3

| Effect of L-tryptophan on the antitussive action of dextromethorphan in rats | | |
|---|---|---|
| Test drug | AtD50* (mg/kg) | |
| | (i.v.) | (i.d.) |
| DM | 1.60 (1.15-2.22)** | 11.43 (7.27-17.97) |
| DM:L-Trp (1:5) | 0.79 (0.50-1.12) | 7.19 (4.85-10.66) |

\* 50% antitussive dose
\*\* 95% confidence limit

## INDUSTRIAL UTILIZABILITY

As stated above, according to the present invention, the effect of the antitussive agent is enhanced, therefore there is obtained the equivalent antitussive activity to the prior antitussive preparation in spite of decreasing the amount of the antitussive agents in the antitussive preparation.

Accordingly, the present invention makes it possible to provide an antitussive preparation which hardly occurs side effect by which the plasticity of physical dependence of the antitussive agent is aggravated.

## Claims

1. An antitussive composition comprising an antitussive agent and L-tryptophan.

2. The antitussive composition according to Claim 1 wherein the amount of L-tryptophan is from 2 to 50 parts by weight relative to one part by weight of the antitussive agent.

3. The antitussive composition according to Claim 1 or 2 wherein the antitussive agent is one or more members selected from the group consisting of dihydrocodeine, noscapine and dextromethorphan.

4. An activity enhancing preparation for an antitussive agent comprising L-tryptophan as an ingredient.

5. The activity enhancing preparation according to Claim 4 wherein the amount of L-tryptophan is from 2 to 50 parts by weight relative to one part by weight of the antitussive agent.

6. The activity enhancing preparation according to Claim 4 or 5 wherein the antitussive agent is one or more members selected from the group consisting of dihydrocodcine, noscapine and dextromethorphan.

7. A kit for the treatment of cough comprising an antitussive agent and L-tryptophan.

8. The kit for the treatment of cough according to Claim 7 wherein the amount of L-tryptophan is from 2 to 50 parts by weight relative to one part by weight of the antitussive agent.

9. The kit for the treatment of cough according to Claim 7 or 8 wherein the antitussive agent is one or more members selected from the group consisting of dihydrocodeine, noscapine and dextromethorphan.

10. A method for the treatment of cough which comprises administering an effective amount of an antitussive agent and L-tryptophan.

11. The method for the treatment of cough according to Claim 10 wherein the effective amount of L-tryptophan is from 2 to 50 parts by weight relative to one part by weight of the antitussive agent.

12. The method for the treatment of cough according to Claim 10 or 11 wherein the antitussive agent is one or more members selected from the group consisting of dihydrocodeine, noscapine and dex-

tromethorphan.

# INTERNATIONAL SEARCH REPORT

International Application No PCT/JP90/00776

## I. CLASSIFICATION OF SUBJECT MATTER (if several classification symbols apply, indicate all) 6

According to International Patent Classification (IPC) or to both National Classification and IPC

Int. Cl⁵    A61K31/485, A61K31/405, A61K45/00

## II. FIELDS SEARCHED

Minimum Documentation Searched 7

| Classification System | Classification Symbols |
|---|---|
| IPC | A61K31/405, A61K31/485, A61K45/00 |

Documentation Searched other than Minimum Documentation
to the Extent that such Documents are Included in the Fields Searched 8

## III. DOCUMENTS CONSIDERED TO BE RELEVANT 9

| Category * | Citation of Document, 11 with indication, where appropriate, of the relevant passages 12 | Relevant to Claim No. 13 |
|---|---|---|
| P | JP, A, 2-56425 (Richardson Vix Inc.), 26 February 1990 (26. 02. 90), (Family: none) | 1-3, 4-6, 7-9 |
| P | JP, A, 2-62823 (Richardson Vix Inc.), 2 March 1990 (02. 03. 90), (Family: none) | 1-3, 4-6, 7-9 |

\* Special categories of cited documents: 10

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

## IV. CERTIFICATION

| Date of the Actual Completion of the International Search | Date of Mailing of this International Search Report |
|---|---|
| August 21, 1990 (21. 08. 90) | September 10, 1990 (10. 09. 90) |

| International Searching Authority | Signature of Authorized Officer |
|---|---|
| Japanese Patent Office | |

**FURTHER INFORMATION CONTINUED FROM THE SECOND SHEET**

**V.☒ OBSERVATIONS WHERE CERTAIN CLAIMS WERE FOUND UNSEARCHABLE** [1]

This international search report has not been established in respect of certain claims under Article 17(2) (a) for the following reasons:

1.☒ Claim numbers 10 ~ 12 because they relate to subject matter not required to be searched by this Authority, namely:

Claims 10 to 12 are relevant to method for disposal of a human body through medical treatment.

2.☐ Claim numbers , because they relate to parts of the international application that do not comply with the prescribed requirements to such an extent that no meaningful international search can be carried out, specifically:

3.☐ Claim numbers , because they are dependent claims and are not drafted in accordance with the second and third sentences of PCT Rule 6.4(a).

**VI.☐ OBSERVATIONS WHERE UNITY OF INVENTION IS LACKING** [2]

This International Searching Authority found multiple inventions in this international application as follows:

1.☐ As all required additional search fees were timely paid by the applicant, this international search report covers all searchable claims of the international application.

2.☐ As only some of the required additional search fees were timely paid by the applicant, this international search report covers only those claims of the international application for which fees were paid, specifically claims:

3.☐ No required additional search fees were timely paid by the applicant. Consequently, this international search report is restricted to the invention first mentioned in the claims; it is covered by claim numbers:

4.☐ As all searchable claims could be searched without effort justifying an additional fee, the International Searching Authority did not invite payment of any additional fee.

Remark on Protest

☐ The additional search fees were accompanied by applicant's protest.

☐ No protest accompanied the payment of additional search fees.

Form PCT/ISA/210 (supplemental sheet (2)) (January 1985)